# EUROPEAN PATENT APPLICATION

(11) **EP 2 213 336 A2**
(43) Date of publication of application: **04.08.2010**
(21) Application number: 10250158.2
(22) Date of filing: 29.01.2010
(51) Int. Cl.: A61Q 17/04, A61K 8/02, A61K 8/03, A61K 8/27, A61K 8/29, A61K 8/34, A61K 8/37, A61K 8/36

(54) **Personal care sunscreen compositions having reduced eye irritation**

(30) Priority: 30.01.2009 US 148561 P
(71) Applicant: Johnson & Johnson Consumer Companies, Inc., Skillman, NJ 08558 (US)
(72) Inventor: Halimi, Laurence, Belle Mead, NJ 08502 (US); Kim, Blair, Hillsborough, NJ 08844 (US)
(74) Representative: Kirsch, Susan Edith

(57) **Abstract**

The present invention is directed to a stable, minimal energy required self-assembling lamellar and spherulite composition comprising: mixture water, fatty alcohol, fatty acid, salt of fatty acid, polyglyceryl fatty acid ester and oils. The present invention relates to composition that can benefit eye mildness, even distribution of sunscreen physical filters on skin and enhanced stability.

## Description

**Related application information:** This application is a continuation-in-part of U.S. Patent Application Serial No. 12/362,929 (Attorney Docket No. JBP5046USNP) filed January 30, 2009 and is also related to U.S. Serial No. 61/148,561, filed January 30, 2009 and U.S. Patent Application Serial No. (Attorney Docket No. JBP5046USNP1), filed January 12, 2010, the subject matter of which is hereby incorporated herein by reference.

### FIELD OF THE INVENTION

This invention relates to self-assembling lamellar and spherulite structures that provide enhanced eye mildness of topical personal care sunscreen products, as well as even distribution of product on the skin as well as enhanced physical stability.

### BACKGROUND OF THE INVENTION

Personal care products that are applied topically to the skin, including facial moisturizers, lotions, creams, shampoos, sunscreens and the like, often come into contact with the human eye. This may occur through direct application due to an individual placing the product on his or her hands, applying the product onto the skin and then inadvertently contacting the eye. Another route that such products may take into the eye is that of perspiration, being carried into the eye when an individual applies the product to the face and sweat carries it into the eye. The effects of such a process include ocular tearing, itching, redness and swelling.

Consumers become exceedingly dissatisfied with sunscreen products during the summer months when their eyes sting or burn due to sunscreen products contacting the eye. Consumers of other types of products, including both leave-on and rinse-off products such as skin creams, cleansers and washes, can experience pain due to the irritating nature of many topically-applied personal care products.

To resolve this problem in sunscreen formulation, sunscreens often contain a water-in-oil vehicle delivery system to promote water resistance and to enhance occlusivity of a product. However, when highly occlusive water-in-oil products are used daily, they can prevent the regeneration of natural lipid barrier of the skin generating more dry skin feel after each use. Typical water-in-oil products' aesthetics often leaves waxy or oily after-feel not suitable for daily use.

Furthermore, there may be a limit on the degree to which water-in-oil vehicles deliver sunscreen efficacy. Sunscreen filters such as titanium dioxide and zinc oxide or any other colloidal particles may be localized mainly in the oil phase of water-in-oil formulations, which may create uneven application of sunscreen filters on skin.

Another problem associated with formulating with metal oxides is the difficulty of establishing formulation stability. Metal oxides, whether hydrophobically or hydrophilically coated, often settle out of solution in sunscreen products due to heterogeneous suspension and/or dispersion.

Other types of formulations, including leave-on and rinse-off formulations, may raise the same types of issues, especially when an active particulate ingredient is included in these formulations. Many creams, emulsions, lotions, as well as shampoos and other cleansers, cause stinging and discomfort when instilled into the eye during use.

U.S. Patent Publication No. US20050238677A1 relates to oil-in-water emulsions wherein an emulsifier blend, comprising a mixture of at least two sucrose esters in combination with at least one solid fatty alcohol, forms a multi-lamellar liquid crystalline network. Making these emulsions requires high shear energy input. Further, the compositions described do not address concerns associated with inorganic sunscreen product instability or mildness to skin and eyes.

U.S. Patent Publication No. US20050265936 relates to a sunscreen formulation comprising a sunscreen, a structurant and an emulsion comprising a structurant and an emulsion comprising a homogenized mixture of wax and alcohol components, at least one of which is a surfactant, wherein the formulation comprises a stable lamellar or spherulite phase. However, the compositions described therein may contain surfactants that are irritating to the eyes, such as sodium lauroamphoacetate, sodium trideceth sulfate and cocamide MEA. These compositions also require additional high shear energy to form the spherulite structures.

It is desirable to provide personal care compositions that can minimize eye irritation while providing an improved efficacy of active delivery and formulation process. The present invention overcomes this problem.

### SUMMARY OF THE INVENTION

This invention relates to personal care compositions having at least two phases and methods of making said compositions that are unexpectedly mild to the eye. The compositions of this invention more particularly relate to compositions containing components that, when combined with each other, and without the application of energy to the composition, assemble themselves into lamellae and a spherulitic G Phase.

The term "G Phase" refers to a liquid crystal lamellar phase, of the type known as "neat phase" or "lamellar phase" in the literature, and its characteristic textures can be identified under a cross-polarized microscope. The terms "lamellae" or "lamellar structure", as used herein, refers to an ordered liquid crystalline phase in which plurality of bilayers are arranged in parallel arrays separated by water layers.

The term "spherulitic G Phase", as used herein, refers to concentric bilayers alternative with aqueous phase with a G phase or expanded G phase spacing, e.g., small, rounded bodies within the composition that may have a radiate fibrous structure. The term also refers to layers of rounded spherical or spheroidal bodies conforming the structure.

Preferably, the compositions of this invention contain water and at least one polyglyceryl fatty acid ester, a fatty acid and a salt of said fatty acid and a sunscreen ingredient. The compositions of this invention may preferably also contain a moisturizing agent, saturated single chain fatty alcohol, polyesters, hydrophobic-natured oils and other personal care or cosmetic ingredients. Preferably, the fatty acid is a saturated, single chain fatty acid. In order to achieve the self-forming structures of the compositions of this invention, the appropriate ratio between the saturated single chain fatty acid and its salt is preferably from about 1:2 to about 1:4. Preferably, in order to achieve the self-forming structures of the compositions of this invention, the appropriate ratio between the polyglyceryl fatty acid ester, the fatty acid and the salt of the fatty acid should be about 1:1:2 or higher.

### BRIEF DESCRIPTION OF THE DRAWINGS

FIGURE 1 is a graph representative of the X-ray diffraction patterns of the compositions described in Example 8.

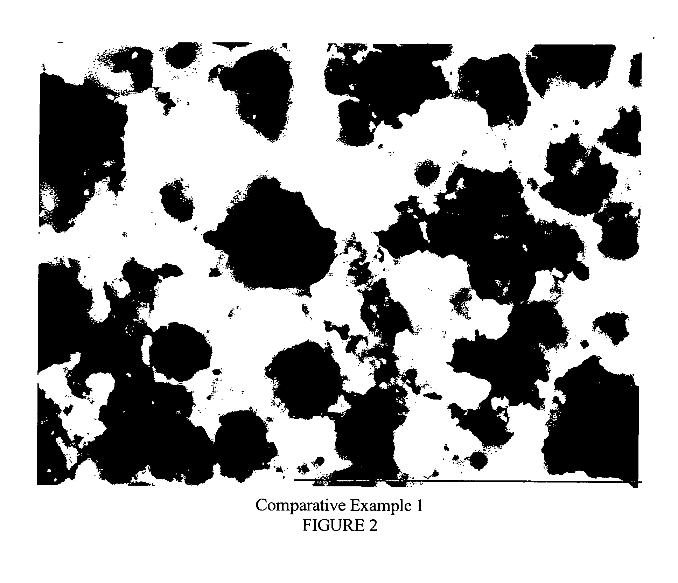

FIGURE 2 is an environmental scanning electron microscopic (ESEM) photograph of a comparative composition described in Example 9.

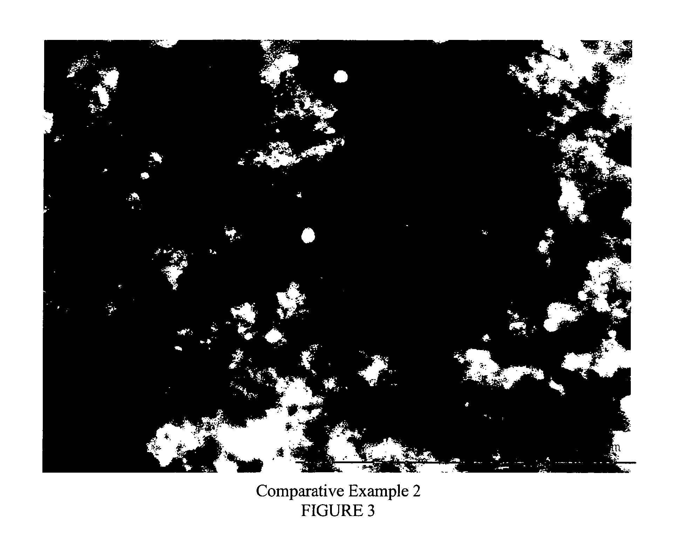

FIGURE 3 is an ESEM photograph of a comparative composition described in Example 9.

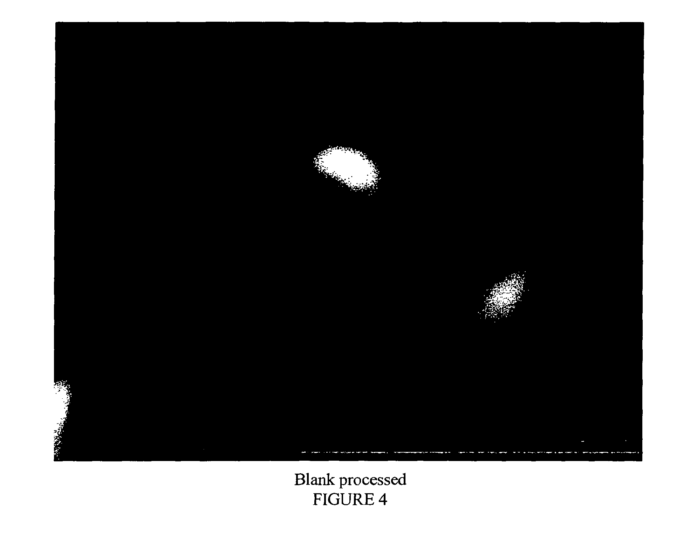

FIGURE 4 is an ESEM photograph of a blank composition described in Example 9.

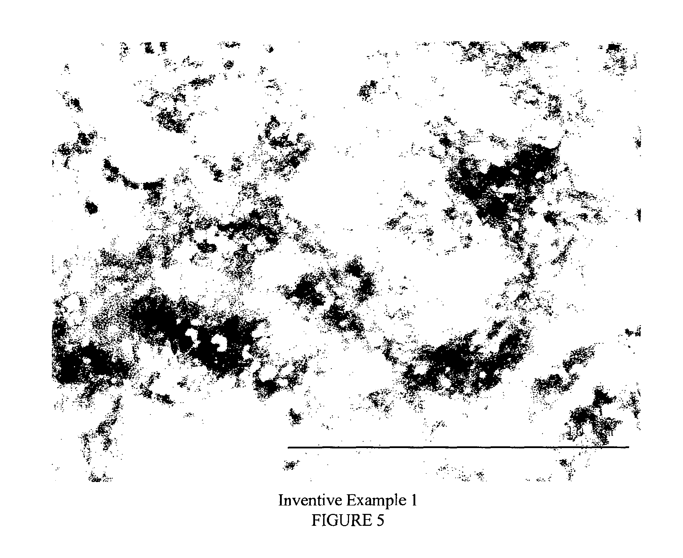

FIGURE 5 is an ESEM photograph of an inventive composition described in Example 9.

### DETAILED DESCRIPTION OF THE PREFERRED EMBODIMENTS

The compositions of this invention preferably are in the form of at least a two-phase composition, more preferably, a water-in-oil structuring blend. They preferably contain at least one polyglyceryl fatty acid ester, fatty acid and a salt of said fatty acid as well as a sunscreen ingredient. These elements, when combined, form self-assembling multi-lamellar and spherulite structures. Salts of fatty acids have unexpectedly been found to produce stable emulsions over a range of viscosities with distinctive aesthetic profiles and rheological characteristics.

More preferably, the compositions of this invention further contain at least one saturated single chain fatty alcohol, at least one hydrophobic-nature oil and water.

The lamellar network of the compositions of this invention masks or "hides" oily and heavy ingredients in the compositions, such as sunscreen and petrolatum, yielding lighter "skin feel" after application. Thus, when applied to the skin, the composition is not oily or heavy and does not present itself as an oily, substantive layer on the skin. The bilayer lamellar and spherulite structure arrangements promote product spreadability across the skin and increase the compatibility of oil with skin, thereby yielding non-greasy and/or non-sticky skin feel.

The oil phase of the compositions of this invention should contain at least one fatty alcohol, at least one polyglyceryl fatty acid ester, at least one fatty acid, at least one metallic salt of fatty acid and at least one hydrophobic-nature oil or mixtures of oils.

The self-assembling personal care compositions of this invention preferably also preferably include at least one polyglyceryl fatty acid ester. Such polyglyceryl fatty acid esters may be saturated or unsaturated and branched. More preferably, the polyglyceryl fatty acid esters utilized in the compositions of this invention should be polyglyceryl esters having from 16 to 22 carbon atoms. More preferably, the polyglyceryl fatty acid esters used in the compositions of this invention should be mono-, di- or tri- polyglcyeryl fatty acid esters. Most preferably, saturated or unsaturated and branched chain fatty acid ester such as polyglyceryl-2 isostearate, polyglyceryl-4 isostearate, polyglyceryl-4 tristearate, polyglyceryl-3 oleate, diglycerol monooleate and diglycerol monostearate and the like.

Preferably, the mono-, di- or tri- polyglyceryl fatty acid esters used in the compositions of this invention should have an HLB value of about 2 to about 8.

The polyglyceryl fatty acid esters should be present in the compositions of this invention in a ratio with respect to the fatty acid component of from about 6:1 to about 1:1.

The self-assembling personal care compositions of this invention also preferably include at least one single-chain solid fatty alcohol having aliphatic hydrocarbons containing about 14 to about 22 carbon atoms with melting points ranging from about 38°C to about 71°C. More preferably, such single-chain solid fatty alcohols have from 16 to about 20 carbon atoms. Most preferably, such single-chain solid fatty alcohols are selected from the group consisting of Cetyl Alcohol, Stearic Alcohol and Arachidyl alcohol. The amount of fatty alcohol in the compositions of the present invention may preferably range from about 0.5% to about 10%, and more preferably from about 1.5% to about 5% by weight of the composition. The precise amount of fatty alcohol will vary depending upon the desired aesthetics, rheology, dispersion and compatibility of components in the compositions of this invention, as well as the delivery of active ingredients chosen and the amount of active ingredient delivery. The fatty alcohol, if present, and the fatty acid are preferably combined in the compositions of this invention in a ratio of about 1:2 to about 1:4 (fatty alcohol:fatty acid).

Preferably, the compositions of this invention should contain a saturated fatty acid with a melting point ranging from about 31°C to about 80°C. More preferably, the fatty acids used in the compositions of this invention should have from about 10 to about 22 carbon atoms. They should be straight-chained fatty acids. Most preferably, the fatty acids useful in the compositions of this invention should include myristic acid, palmitic acid, stearic acid and arachidic acid and the like. The most preferred fatty acid is stearic acid.

The oil phase of the compositions of this invention should also contain at least one oil. As used herein, the term "oil" is a hydrophobic material that can aid in balancing the intermolecular forces to form micelle aggregates or to limit their sizes. Oils also serve as emollient ingredients to benefit product spreadability, skin feel and delivery of hydrophobic active ingredients such as but not limited to, Vitamins D, E, K and A, and sunscreen filters. Oils that are useful in the compositions of this invention include a variety of hydrocarbon-based oil, silicones, fatty acid derivatives, glycerides, vegetable oils, vegetable oil derivatives, alkyl esters, wax esters, beeswax derivatives, sterols, and phospholipids and combinations thereof ranging from approximately 20% to 50%, based on the total weight of the composition.

Suitable hydrocarbon oils for preferable use in the compositions and methods of this invention include petrolatum, mineral oil, micro-crystalline waxes, squalene and combinations thereof. The example of silicone oils suitable for use as hydrophobic materials for this invention include dimethicone, dimethiconol, phenyl dimethicone and cyclic polysiloxanes and combinations thereof. Silicone oils having viscosities from about 0.5 to about 100,000 centistokes at 25°C may also be useful in the composition.

Glycerides useful in the compositions of this invention include castor oil, sunflower seed oil, coconut oil and derivatives, vegetable oils and derivatives, palm oil, jojoba oil, shea butter, lanolin and combinations thereof.

Alkyl ester oils including, but not limited to isopropyl esters of fatty acids and esters of long chain fatty acids may also be suitable for use in the compositions of this invention. More preferably, the following alkyl esters may be useful in the compositions of this invention: isopropyl palmitate, isopropyl myristate, myristyl myristate, isohexyl palmitate, decyl oleate, isononyl isononanoate and a combination thereof.

Hair or skin conditioning agents may be used in the compositions of this invention, including, but not limited humectants, protein and protein derivatives such as wheat protein, rice protein and milk protein, silicone conditioning agents and lipids and combination thereof. A variety of these materials can be employed either in the oil phase of the compositions or in their water phase depending upon their hydrophobicity. Preferably, such conditioning agents should be present in the compositions of this invention in amount of from about 0.01% to about 30%, more preferably from about 0.1% to about 20%, and most preferably from about 0.5% to about 10% by weight of the composition.

The aqueous phase of the compositions of this invention is preferably present in amounts of from about 30% to about 50% by weight relative to the total weight of the composition. The aqueous phase preferably contains water, or a combination of water and at least one polyhydric alcohol. Preferably, such polyhydric alcohols may be chosen from the group consisting of glycols, glycerols, polyethylene glycol, propylene glycol, other water soluble ingredients and the like. The compositions of this invention may also preferably include small percentages of thickening agents including polymer for the aqueous phase, from about 0.01% to about 0.5% weight/weight, including cellulose and/or carbomer family well known in the art. Preferably, the water phase of the compositions of this invention should have a pH of at least 5.

Thickeners are preferably selected from the group consisting of inorganic water thickeners, charged polymeric materials, water soluble polymers and mixtures thereof. Inorganic water thickeners preferably include ingredients selected from the group consisting of silicas, clays such as laponite, modified starches and mixtures thereof. Charged polymeric water thickeners preferably include acrylates/C10-30 alkyl acrylate crosspolymer, carbomers, ammonium acryloyldimethyltaurate/vp copolymer, ammonium acryloyldimethyltaurate, polyacrylamide and mixtures thereof. Water-soluble polymers preferably include cellulosic gels, hydroxylpropyl starch phosphates and mixture thereof.

When present, hydrophilic thickening agents assist in suspending and/or effectively delivering hydrophilic ingredients onto the skin or scalp. The aqueous phase should preferably have a pH of from about 5 to about 8, more preferably about 6 to about 7. The appropriate pH maybe achieved by utilizing pH adjusters to facilitate the proper pH range.

The oil phase of the compositions of this invention should also preferably include at least one electrolyte compound. Such electrolyte compound should preferably include at least one fatty acid salt containing alkali or alkaline earth metal ions, including bivalent metal ions such as magnesium, calcium, ammonium and mixtures thereof and the like. More preferably, such electrolyte should be magnesium stearate. Preferably, the electrolyte compounds useful in the compositions of this invention should be added to the oil phase of the composition. The amount of electrolyte in the compositions of this invention preferably range from about 0.1% to about 20%, based on the total weight of the composition. More preferably, they should be present in an amount of from about 1% to about 10% by weight of the composition. The respective fatty acid and the metal ions are typically combined in a ratio of from about 1: 4 to about 1:2. Preferred saturated fatty acids have between 10 and 22 carbons described above.

Without being bound to any particular theory, it is thought that the self-assembling nature of the compositions of this invention relies upon the following structural and chemical relationships. Metallic salts of long chain fatty acid are molecules composed of charged ions and a hydrophobic tail with an ionic bond that prevents separation between the metal ion and long chain hydrocarbon tail. As a unit, the metallic salts of long chain fatty acids strongly influence closer packing of the overall structure and a higher degree of crystalline order of the compositions in that there is less tilting of hydrocarbon chains, which decreases the area taken up by each molecule.

Correlation between the metallic salt of fatty acid and its phase behavior can be understood by a well-known geometric model: the critical packing parameter. In the compositions of this invention, the metallic salt of fatty acid provides an important role in forming a self-assembling structure by contributing a "critical packing factor" for the structurants (including the fatty acid, fatty alcohol, hydrophobic oil and fatty acid ester ingredients) close to 1. The "critical packing parameter" is defined as follows: v/aolc, wherein ao=area of headgroup, v=volume of hydrophobic tail and lc=length of hydrophobic tail.

According to this model, the length of the hydrophobic tail of the overall structure needs to be approximately about 4/3 of that of a micelle radius to form a lamellar structure. The metallic salt fatty acid provides this extra length needed to form spontaneous two-dimensional structure. Furthermore, unlike amphiphile molecules that can be separated depending on hydrophobic or hydrophilic environment, the strong ionic bond between the metallic head group and hydrocarbon tail prevents the separation while increasing the lamellar bilayer stability.

The sunscreen active ingredients suitable for use in the compositions of this invention preferably include physical sunscreens such as titanium dioxide and zinc oxide. These metal oxides should have a particle size ranging from about 10 nm to about 100 nm. Metal oxides that are useful in the compositions of this invention may be coated with dimethicone, alkoxy titanates, methyl polysiloxanes, silica and/or alumina or mixture thereof. A wide variety of chemical sunscreen actives are suitable for use in this invention including main chemistry classes of UV filters: PABA and p-aminobenzoates, Salicylates, Cinnamates, Benzophenones, Anthranilates, Dibenzoyl methanens, camphor derivatives and mixtures thereof and the like. The appropriate amount of sunscreen present in the compositions of this invention will vary depending on the types of sunscreen filters chosen and the desired UVB and/or UVA values.

Additional ingredients that may be incorporated into the compositions of this invention includes preservatives, antimicrobial and antifungal actives that are capable of destroying microbes, preventing the development of microbes or preventing the pathogenic action of microbes. These may be present in amounts effective to prevent microbe growth in the compositions of this invention so as to preserve their microbiological stability over long periods of time and under different environmental conditions.

Preferably, antimicrobial or antifungal actives may be present in the compositions of this invention in amounts of about 0.001% to about 5.0% by weight of the composition. Most preferably, such actives may be present in the compositions of this invention in the amount of about 0.1% to about 2% by weight of the composition. Such antimicrobial and antifungal active ingredients include preferably, phenoxyethanol, parabens, methylisothizolinone, chlorophenesin and the like. In addition, hydrocortisone, tetracycline, ibuprofen, naproxen, acetaminophen, benzoylperoxide, salicylic acid, lipoic acid, pyrithione zinc, lidocanehydrochloride, clotrimazole, arachidonic acid and mixture thereof may be included in the compositions of this invention.

It is believed that the compositions of this invention may also be used to provide eye mildness to surfactant-containing compositions such as washes and shampoos. Such compositions may contain, in addition to fatty acids, the salt of said fatty acid and polyglyceryl fatty acid ester, surfactant ingredients including, but not limited to the following.

In one embodiment, the composition contains one or more surfactants. In one embodiment, the composition contains a lathering surfactant. What is meant by a "lathering surfactant" is a surfactant that generates lather when combined with water and mechanically agitated. In one embodiment, the lathering surfactant has an initial foam height reading of at least 20 mm, such as at least 50 mm, in the Standard Test Method for Foaming Properties of Surface-Active Agents D1173-53 Set forth in the ASTM Annual Book of ASTM Standards 1001 Section 15 Volume 15.04 (using a concentration of 5 grams per liter, temperature of 49°C, and water hardness of 8 grains per gallon). Examples of lathering surfactants include, but are not limited to, anionic, nonionic, cationic, and amphoteric lathering surfactants.

Nonlimiting examples of anionic lathering surfactants include those selected from the group consisting of sarcosinates, sulfates, isethionates, taurates, phosphates, lactylates, and glutamates. Specific examples include, but are not limited to, those selected from the group consisting of sodium lauryl sulfate, ammonium lauryl sulfate, ammonium laureth sulfate, sodium laureth sulfate, sodium trideceth sulfate, ammonium cetyl sulfate, sodium cetyl sulfate, ammonium cocoyl isethionate, sodium lauroyl isethionate, sodium lauroyl lactylate, triethanolamine lauroyl lactylate, sodium caproyl lactylate, sodium lauroyl sarcosinate, sodium myristoyl sarcosinate, sodium cocoyl sarcosinate, sodium lauroyl methyl taurate, sodium cocoyl methyl taurate, sodium lauroyl glutamate, sodium myristoyl glutamate, and sodium cocoyl glutamate and mixtures thereof

Nonlimiting examples of nonionic lathering surfactants include those selected from the group consisting of alkyl glucosides, alkyl polyglucosides, polyhydroxy fatty acid amides, alkoxylated fatty acid esters, lathering sucrose esters, amine oxides, and mixtures thereof. Specific examples include, but are not limited to, nonionic surfactants to those selected form the group consisting of C8-C14 glucose amides, C8- C14 alkyl polyglucosides, sucrose cocoate, sucrose laurate, lauramine oxide, cocoamine oxide, and mixtures thereof.

Nonlimiting examples of amphoteric lathering surfactants (which also includes zwitterionic lathering surfactants are those selected from the group consisting of betaines, sultaines, hydroxysultaines, alkyliminoacetates, iminodialkanoates, aminoalkanoates, and mixtures thereof.

Nonlimiting examples of amphoteric surfactants of the present invention include disodium lauroamphodiacetate, sodium lauroamphoacetate, cetyl dimethyl betaine, cocoamidopropyl betaine, cocoamidopropyl hydroxy sultaine, and mixtures thereof.

In the formulation of the sunscreen composition of the present invention, it was discovered that the invention sunscreen composition, which includes inorganic sunscreen filters exhibits substantial phase stabilities over time or lack of syneresis. For example, samples were placed in 40°C, 50°C and 60°C oven for duration of 6 months without any syneresis or phase separation while maintaining constant rheological properties. A method of making a composition in accordance with this invention includes the following steps: the oil phase should be prepared by mixing at least one fatty acid, at least one metallic fatty acid salt and at least one polyglyceryl fatty acid ester. Optionally, the oil phase may more preferably contain at least one fatty alcohol, and hydrocarbon oils and/or glycerides. The mixture is then heated to the melting temperature of about 60° to about 75°C. It is important that the temperature be kept lower than the melting point of the fatty acid and fatty alcohol components of the compositions of this invention. Separately, a water phase is prepared by combining thickeners, preservatives and moisturizing agents. Thickeners are preferably selected from the group consisting of inorganic water thickeners, charged polymeric materials, water soluble polymers and mixtures thereof. Inorganic water thickeners suitable for use in the compositions of this invention include silicas, clays such as Laponite, modified starched or mixture thereof. Polymeric water thickeners include acrylates/C10-30 alkyl acrylate crosspolymer, carbomers, ammonium acryloyldimethyltaurate/vp copolymer, ammonium acryloyldimethyltaurate, polyacrylamide, carbopols or mixtures thereof. Water soluble polymers may include polymers such as celluloisic gel, hydroxylpropyl starch phosphate and mixtures thereof.

The water phase is then added to the oil phase with mixing. Inorganic sun filters may then be added to the composition as sunscreen active ingredients. Preferably, such inorganic sun filters are predispersed in oils, glycerides and/or fatty acid derivatives separately before adding to the remaining oil phase ingredients in order to assure uniform dispersion of the particles.

Preferably, using the distribution method set forth below in Example 9, the particles of sunscreen contained in the compositions of this invention are distributed such that their Distribution Index is at least 70%, more preferably at least about 80%.

The following examples serve to exemplify the compositions and methods of this invention, but should not be considered to limit the scope or breadth of the invention in any way.

### Example 1:

A composition in accordance with this invention was made as follows: The following ingredients were added to a container one at a time; polyglyceryl-4 isostearate, cetyl alcohol, stearic acid, magnesium stearate, oils. The ingredients were heated to between 60°C and 75°C while stirring. In a separate beaker, add water, thickener and when hydrated, and pH adjuster added to achieve a pH of about 6 to about 7. Another thickener, modified hydroxyethylcellulose was added to the mixture while stirring. When uniform, humectant and preservatives were added while heating to 60-75°C. The water phase was then added into the oil phase while mixing. The composition was cooled to room temperature.

The following base formulation was created using the same process as set forth above with the following ingredients.

### Formula A

| **Ingredient** | **Formula 1** |
|---|---|
| Water | q.s |
| Thickener | 0.07 |
| pH adjuster | 0.03 |
| Humectant | 3 |
| Preservative | 0.6 |
| Polyglyceryl-4 isostearate | 6.6 |
| Cetyl Alcohol | 1.65 |
| Stearic Acid | 3.3 |
| Magnesium Stearate | 6.6 |
| Oil | 34 |

### Example 2: Sunscreen Formulations Sunscreen formulations may be created using the same process as set forth above with the following ingredients.

| Phase | Ingredient | %w/w |
|---|---|---|
| Water | Water | q.s |
| | Thickener | 0.02-0.09 |
| | Humectant | 1-3 |
| | Preservatives | 0.5-0.6 |
| PH adjuster | Sodium Hydroxide | q.s to pH6-7 |
| Oil | Fatty acid ester | 1-6.6 |
| | Fatty Alcohol | 0.5-1.65 |
| | Fatty Acid | 1-3.3 |
| | Fatty acid salt | 2-6.6 |
| | Oil | 22-34 |
| Sunscreen | Titanium Dioxide | 8-11 |
| Filter | | |
| | Zinc Oxide | 4-5 |

### Example 3: Sunscreen with Fragrance Oil

The following lotion formulations may be created using the same process as set forth in Example 1 with the following ingredients.

| Phase | Ingredient | %w/w |
|---|---|---|
| Water | Water | q.s |
| | Thickener | 0.02-0.09 |
| | Humectant | 1-3 |
| | Preservatives | 0.5-0.6 |
| PH adjuster | Sodium Hydroxide | q.s to pH6-7 |
| Oil | Fatty acid ester | 1-6.6 |
| | Fatty Alcohol | 0.5-1.65 |
| | Stearic acid | 1-3.3 |
| | Fatty acid salt | 2-6.6 |
| | Oil | 21.8-34 |
| Sunscreen | Titanium Dioxide | 8-11 |
| Filter | | |
| | Zinc Oxide | 4-5 |
| Fragrance | Fragrance | 0.2 |

### Example 4: Sunscreen with active oil

The following lotion formulations may be created using the same process as set forth in Example 1 with the following ingredients.

| Phase | Ingredient | %w/w |
|---|---|---|
| Water | Water | q.s |
| | Thickener | 0.02-0.09 |
| | Humectant | 1-3 |
| | Preservatives | 0.5-0.6 |
| PH adjuster | Sodium Hydroxide | q.s to pH6-7 |
| | Fatty acid ester | 1-6.6 |
| | Fatty Alcohol | 0.5-1.65 |
| | Stearic acid | 1-3.3 |
| | Fatty acid salt | 2-6.6 |
| | Oil | 21.8-34 |
| Sunscreen | Titanium Dioxide | 8-11 |
| Filter | | |
| | Zinc Oxide | 4-5 |
| Fragrance | Fragrance | 0.2 |
| Active oil | Active oil | 0.1-1 |

### Example 5:

The following skin cream formulation may be created using the same process as set forth in Example 1 with the following ingredients.

### Example 2. Skin cream

| Phase | Ingredient | %w/w |
|---|---|---|
| Water | Water | q.s |
| | Carbomer | 0.04 |
| | Glycerin | 5 |
| | Preservatives | 0.8 |
| PH adjuster | Sodium Hydroxide | q.s to pH6-7 |
| Oil | Polyglyceryl-4 isostearate | 3.2 |
| | Cetyl alcohol | 0.8 |
| | Stearic acid | 1.6 |
| | Magnesium stearate | 3.2 |
| | C18-C36 acid triglyceride | 3 |
| | Microcrystalline wax | 5 |
| | Isodecyl Neopentanoate | 7 |
| | Isopropyl Myristate | 8 |
| | Dimethicone | 1 |

### Example 6: Baby Lotion

The following lotion formulation may be created using the same process as set forth in Example 1 with the following ingredients.

### Baby Lotion

| Phase | Ingredient | %w/w |
|---|---|---|
| Water | Water | q.s |
| | Hydroxyethylcellulose | 0.2 |
| | Glycerin | 5 |
| | Phenoxyethanol | 0.5 |
| | Methyl paraben | 0.2 |
| | Ethyl paraben | 0.1 |
| Oil | Polyglyceryl-4 | 3.6 |
| | isostearate | |
| | Cetyl alcohol | 0.9 |
| | Stearic acid | 1.8 |
| | Magnesium stearate | 3.6 |
| | Ethylhexyl palmitate | 3 |
| | Petrolatum | 6 |
| | Glyceryl Stearate | 2 |
| | Isopropyl Myristate | 8 |
| | Dimethicone | 1 |

### Example 7: Assessment of Physical Structure of Compositions

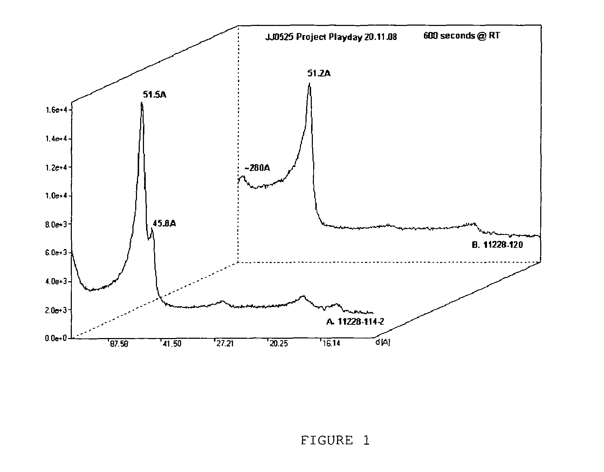

In order to assess the presence of lamellar and spherulite structure, the formula was examined by use of small-angle X-ray diffraction (SAXS) and cryo-Scanning Electron Microscopy (cryo-SEM). The SAXS scattering pattern of the sample illustrates the existence of two lamellar phases, one being spherulitic G phase in nature in its peak and intensity, with d spacing of 51.5A° and 45.8 A° respectively. The relationship between the first order peak and the low intensity peaks, second, third and fourth confirms lamellar structure. This pattern is reflected in Figure 1.

### Example 8: Assessment of Eye Mildness

An *in vitro* method using the cornea model Epi-Ocular was used to assess eye mildness of the compositions of this invention. The Epi-Ocular model is a cornea model derived from human keratinocytes which is then treated with the test item form 3, 30 minutes and 60 minutes. The cell viability is then measured quantitatively after extraction from tissue. The time point when 50% of the cells are dead (ET50) which allows the classification of the tested item, is calculated. The passing criterion of Epi-ocular test result is the time point of ET50 is greater than or equal to 24 hours.

**Table.1 Test Items**

| No. | Item |
|---|---|
| 1 | Formula A |
| 2 | Formula A with physical sunfilters, TiO2 and ZnO |
| 3 | Formula A with fragrance |
| 4 | Formula A with active oil |

**Table.2 Result**

| No. | Item |
|---|---|
| 1 | ET50 >/= 24 hrs --mild |
| 2 | ET50 >/= 24 hrs--mild |
| 3 | ET50 >/= 24 hrs--mild |
| 4 | ET50 >/= 24 hrs--mild |

### Example 9: Assessment of Filter Particle

### Distribution

The purpose of this example is to show the distribution of the filter particles as they would be if the composition was spread onto a substrate or skin surface. As previous studies such as those described in Teichmann et al. (J. Biomed. Opt., Vol. 11, 064005 (2006)) show distribution as vertical to the skin surface, a method showing lateral distribution was developed.

In order to evaluate the spreadability and particle distribution of inventive and comparative sunscreen formulations, environmental scanning electron microscopy (ESEM) was used to visualize inorganic sunscreen particle distribution. The ESEM instrument used was a TM-1000 Tabletop Microscope (Hitachi High Technologies America Inc, Schaumburg IL). Collection parameters were as follows: accelerating voltage 15,000v, magnification 10,000x, emission current 40.8 mA, scan speed slow3, image size 1280x1040 pixels. Films of sunscreen formulations were created as follows: 3 50 uL drops of formulation were dispensed in a column pattern onto hydrated VITRO-SKIN (IMS Inc, Portland ME); the drops were spread into a continuous film using a drawdown bar set with a gap size of 500 um; small strips of the film were cut and mounted for ESEM analysis. After drying overnight, ESEM micrographs of the samples were collected at 10,000 times magnification of the sunscreen film samples and of blank VITRO-SKIN controls. The Micrographs were analyzed in Adobe Photoshop 7.0 or Adobe Photoshop 11 (Adobe Systems Incorporated, San Jose CA) using the following procedure: for each sample, threshold intensity was generated by averaging the 97^{th} percentile intensity (from histogram) of three 300x300 pixel regions on the VITRO-SKIN control. Using the calculated threshold, a black and white image was generated using the threshold function where the white areas are brighter than the control and the black areas are darker than the control. The 'Distribution Index' is the percentage of white pixels in the binary image. Formulations tested were as follows: Formula A with 17.17% inorganic UV filter particles (Inventive Example 1), Burt's Bees Chemical Free Sunscreen SPF 15 (Burt's Bees, Durham NC)(Comparative Example 1), and California Baby SPF 18 Moisturizing Sunscreen No Fragrance (California Baby, Los Angeles CA)(Comparative Example 2). The comparative examples were chosen as the filter particles used to provide sunscreen protection were inorganic particles. Untreated Vitro Skin was also analyzed and is referred to the "Blank". All samples were tested in triplicate.

| | Average Distribution Index | Standard Deviation of Distribution Index |
|---|---|---|
| Formula A | 83.3 | 11.6 |
| Comparative 1 | 45.4 | 11.1 |
| Blank | 2.3 | 1.4 |
| Comparative 2 | 8.9 | 8.3 |

As shown in the Figures and data above, the Inventive Example 1 provides filter particle distribution of at least 70%. The Comparative Examples fail to show such a distribution. An even distribution of particles would provide uniform and complete skin coverage, thereby providing better protection from ultraviolet radiation over a larger proportion of exposed skin.

## Claims

1. A topical sunscreen composition having at least two phases which is a self-forming lamellar and spherulite structure comprising: at least one polyglyceryl fatty acid ester, at least one fatty acid, and at least one metallic fatty acid salt, at least one sunscreen and a water phase.

2. A composition according to claim 1 wherein said fatty acid and said metallic fatty acid salt are present in the composition in a weight ratio of from about 1:2 to about 1:4.

3. A composition according to claim 1 wherein said fatty acid, said polyglyceryl fatty acid ester and said metallic fatty acid salt are present in the composition in a weight ratio of about 1:1:2.

4. A composition according to claim 1 or claim 2 wherein said polyglyceryl fatty acid ester and said fatty acid are present in said composition in a weight ratio of from about 6:1 to about 1:1.

5. A composition according to any preceding claim wherein said composition further comprises a fatty alcohol, preferably a straight-chained fatty alcohol having from 14 to 22 carbon atoms.

6. A composition according to any preceding claim wherein said fatty acid is a straight-chained fatty acid having from 10 to 22 carbon atoms, preferably selected from the group consisting of myristic acid, palmitic acid, stearic acid and arachidic acid.

7. A composition according to claim 6 wherein said fatty acid is stearic acid.

8. A composition according to any preceding claim wherein said salt of said fatty acid is selected from the group consisting of alkali and alkaline metal salts.

9. A composition according to claim 8 wherein said salt of said fatty acid is an alkaline metal salt, preferably selected from magnesium, calcium, ammonium and mixtures thereof.

10. A composition according to any preceding claim wherein said water phase has a pH of at least 5.

11. A composition according to any preceding claim wherein said composition further comprises at least one oil, preferably selected from the group consisting of Vitamins D, E, K and A, sunscreen filters, hydrocarbon-based oil, silicones, fatty acid derivatives, glycerides, vegetable oils, vegetable oil derivatives, alkyl esters, wax esters, beeswax derivatives, sterols, and phospholipids and mixtures thereof.

12. A composition according to any preceding claim wherein said composition further comprises at least one polyhydric alcohol.

13. A composition according to any preceding claim wherein said composition is physically stable for at least 60 days at 60°C.

14. A composition according to any preceding claim wherein said sunscreen component is an organic sunscreen, preferably selected from the group consisting of PABA and p-aminobenzoates, Salicylates, Cinnamates, Benzophenones, Anthranilates, Dibenzoyl methanens, camphor derivatives and mixtures thereof.

15. A sunscreen composition according to any of claims 1 to 13 wherein said sunscreen component is an inorganic sunscreen, preferably selected from the group consisting of zinc oxide, titanium dioxide and mixtures thereof, and more preferably imparting a filter distribution of at least 70%.
